# EUROPÄISCHE PATENTANMELDUNG

(11) **EP 1 721 601 A1**
(43) Veröffentlichungstag der Anmeldung: **15.11.2006**
(21) Anmeldenummer: 06009198.0
(22) Anmeldetag: 04.05.2006
(51) Int. Cl.: A61K 8/44, A61Q 19/00, A61K 31/195, A61P 17/00

(54) **Verwendung von Taurin zur Steigerung der epidermalen Lipidsynthese**

(30) Priorität: 11.05.2005 DE 102005022625
(71) Anmelder: Henkel Kommanditgesellschaft auf Aktien, 40589 Düsseldorf (DE)
(72) Erfinder: Döring, Thomas, 41542 Dormagen (DE); Waldmann-Laue, Marianne, 40789 Monheim (DE); Wadle, Armin, 40699 Erkrath (DE); Anderheggen, Bernd, 42781 Haan (DE)

(57) **Zusammenfassung**

Gegenstand der vorliegenden Erfindung ist die Verwendung von Taurin zur Stimulierung der epidermalen Synthese von Barrierelipiden.

## Beschreibung

Die Haut ist das größte Organ des Menschen. Unter ihren vielen Funktionen (beispielsweise zur Wärmeregulation und als Sinnesorgan) ist die Barrierefunktion, diejenige die das Austrocknen der Haut (und damit letztlich des gesamten Organismus) verhindert, die wohl wichtigste. Gleichzeitig wirkt die Haut als Schutzeinrichtung gegen das Eindringen und die Aufnahme von außen kommender Stoffe. Bewirkt wird diese Barrierefunktion durch die Epidermis, welche als äußerste Schicht die eigentliche Schutzhülle gegenüber der Umwelt bildet. Mit etwa einem Zehntel der Gesamtdicke ist sie gleichzeitig die dünnste Schicht der Haut.

Die Epidermis ist ein stratifiziertes Gewebe, in dem die äußere Schicht, die Hornschicht (stratum corneum) den für die Barrierefunktion bedeutenden Teil darstellt.

Sie wird im Kontakt mit der Umwelt abgenutzt und befindet deshalb sich in einem ständigen Erneuerungsprozess, wobei nach außen kontinuierlich feine Schuppen abgegeben und von innen verhorntes Zell- und Lipidmaterial nachproduziert wird.

Das heute in der Fachwelt anerkannte Hautmodell von Elias (P. M. Elias, Structure and Function of the Stratum Corneum Permeability Barrier, Drug Dev. Res. 13, 1988, 97 - 105) beschreibt die Hornschicht als Zwei-Komponenten-System, ähnlich einer Ziegelsteinmauer (Ziegelstein-Mörtel-Modell). In diesem Modell entsprechen die Hornzellen (Corneozyten) den Ziegelsteinen, die komplex zusammengesetzte Lipidmembran in den Interzellularräumen entspricht dem Mörtel. Dieses System stellt im wesentlichen eine physikalische Barriere gegen hydrophile Substanzen dar, kann aber aufgrund seiner engen und mehrschichtigen Struktur gleichermaßen auch von lipophilen Substanzen nur schwer passiert werden. Die besondere Struktur der Hornschicht schützt einerseits die Haut und stabilisiert andererseits ihre eigene Flexibilität durch Bindung einer definierten Wassermenge.

Auch mechanische Belastungen, wie beispielsweise Druck-, Stoß- oder Scherkräfte, können in erstaunlichem Maße durch die Hornschicht allein oder im Verbund mit den tieferen Hautschichten abgefangen werden. Größere Druck-, Dreh- oder Scherkräfte werden über die Verzahnung der Epidermis mit dem Corium an tiefere Hautschichten weitergegeben. Die Regulation des Wasser- und Feuchtigkeitsgehaltes ist eine der wichtigsten Funktionen der epidermalen Lipidmembran. Allerdings hat sie nicht nur eine Barrierewirkung gegen externe chemische und physikalische Einflüsse, sondern trägt auch zum Zusammenhalt der Hornschicht bei.

Die Lipide der Hornschicht bestehen im wesentlichen aus Ceramiden, freien Fettsäuren, Cholesterin sowie Cholesterinsulfat und sind über die gesamte Hornschicht verteilt. Die Zusammensetzung dieser Lipide ist für die intakte Funktion der epidermalen Barriere und damit für die Wasserundurchlässigkeit der Haut von entscheidender Bedeutung.

Bereits bei einer Reinigung der Haut mit Hilfe eines einfachen Wasserbads - ohne Zusatz von Tensiden - kommt es zunächst zu einer Quellung der Hornschicht der Haut. Der Grad dieser Quellung hängt u. a. von der Dauer des Bads und dessen Temperatur ab. Gleichzeitig werden wasserlösliche Stoffe ab- bzw. ausgewaschen, wie z. B. wasserlösliche Schmutzbestandteile, aber auch hauteigene Stoffe, die für das Wasserbindungsvermögen der Hornschicht verantwortlich sind. Durch hauteigene oberflächenaktive Stoffe werden außerdem auch Hautfette in gewissem Ausmaß gelöst und ausgewaschen. Dies bedingt nach anfänglicher Quellung eine nachfolgende Austrocknung der Haut, die durch waschaktive Zusätze noch deutlich verstärkt werden kann.

Bei gesunder Haut sind diese Vorgänge im allgemeinen belanglos, da die Schutzmechanismen der Haut solche leichten Störungen der oberen Hautschichten ohne weiteres kompensieren können. Aber bereits im Fall nicht-pathologischer Abweichungen vom Normalstatus, z. B. durch umweltbedingte Abnutzungsschäden bzw. Irritationen, Lichtschäden, Altershaut usw., ist der Schutzmechanismus an der Hautoberfläche gestört. Bei alter Haut beispielsweise erfolgt die regenerative Erneuerung verlangsamt, wobei insbesondere das Wasserbindungsvermögen der Hornschicht nachlässt. Sie wird deshalb inflexibel, trocken und rissig ("physiologisch" trockene Haut). Ein Barriereschaden ist die Folge. Die Haut wird anfällig für negative Umwelteinflüsse wie die Invasion von Mikroorganismen, Toxinen und Allergenen. Als Folge kann es sogar zu toxischen oder allergischen Hautreaktionen kommen. Trockene Haut weist einen Mangel an Barrierelipiden (Ceramide, Cholesterol und freie Fettsäuren) in der Hornschicht auf. Die verminderte Integrität und Reparaturleistung der Hautbarriere führt zu einem gesteigerten transepidermalen Wasserverlust. Die Haut wird rau, trocken, schuppig und neigt zu Spannungsgefühlen sowie Juckreiz.

Bei pathologisch trockener und empfindlicher Haut oder bei von atopischer Dermatitis betroffener Haut liegt ein Barriereschaden a priori vor. Epidermale Interzellularlipide werden fehlerhaft oder in ungenügender Menge bzw. Zusammensetzung gebildet. Die Konsequenz ist eine erhöhte Durchlässigkeit der Hornschicht und ein unzureichender Schutz der Haut vor Verlust an hygroskopischen Substanzen und Wasser.

Im Stand der Technik sind diverse Hautpflegeprodukte bekannt, die in der Lage sind, die epidermale Barrierefunktion zu verbessern. Diese Hautpflegeprodukte sind jedoch in der Regel okklusiver Natur, z. B. Vaseline und Vaseline-haltige Cremes, und weisen häufig ein klebriges Hautgefühl und/oder einen unangenehmen Tragekomfort auf. Die Salbe oder Creme stellt sozusagen eine (zweite) künstliche Barriere dar, die den Wasserverlust der Haut verhindern soll. Entsprechend leicht kann diese physikalische Barriere - beispielsweise mit Reinigungsmitteln - wieder entfernt werden, wodurch der ursprüngliche, beeinträchtigte Zustand wieder erreicht wird. Darüber hinaus kann die Hautpflegewirkung bei regelmäßiger Behandlung nachlassen. Nach dem Absetzen der Produktanwendung kehrt die Haut sehr schnell wieder in den Zustand vor Behandlungsbeginn zurück. Eine nachhaltige Produktwirkung wird in der Regel also nicht oder nur in einem eingeschränkten Maße erreicht. Um die Haut bei ihrer natürlichen Regeneration zu unterstützen und ihre physiologische Funktion zu stärken, werden den topischen Präparaten in neuerer Zeit zunehmend Interzellularlipidmischungen, wie Ceramide oder Ceramidanaloga, zugesetzt, die von der Haut zum Wiederaufbau der natürlichen Barriere verwendet werden sollen. Allerdings handelt es sich bei diesen Lipiden zumeist um sehr teure Rohstoffe, die außerdem aufgrund ihres hohen Schmelzpunktes schwierig zu formulieren sind. Zudem ist ihre Wirkung begrenzt, da die topisch applizierten Lipide nur in geringem Umfang in die barriererelevanten Lipidmembranen im mittleren Stratum corneum penetrieren und nur zu einem geringen Teil in diese Strukturen integriert werden.

Dem Stand der Technik mangelt es demnach an Zubereitungen, die die Barrierefunktion und die Hydratation der Hornschicht positiv beeinflussen und die physikalisch-chemischen Eigenschaften der Hornschicht und insbesondere der Lamellen aus Interzellularlipiden stärken bzw. sogar wiederherstellen.

Jüngere Untersuchungen haben gezeigt, dass unter trockenen Bedingungen der organische Osmolyt Taurin in der äußeren Granularkeratinozytenschicht akkumuliert wird (J. Invest. Dermatol. 121, 354 - 361 (2003)). Weiterhin wurde gezeigt, dass unter trockenen Bedingungen oder unter UV-Bestrahlung Mechanismen der Barrierereparatur stimuliert werden (J. Invest. Dermatol. 111, 858 - 863 (1998), J. Invest. Dermatol. 109, 783 - 787 (1997), J. Lipid Res. 32, 1151 - 1158 (1991)).

Im Rahmen der vorliegenden Erfindung stellte sich die Frage, inwieweit diese beiden unterschiedlichen epidermalen Reaktionen auf Umwelteinflüsse miteinander im Zusammenhang stehen. Dazu wurde durch wiederholte Behandlung der Haut mit Natriumdodecylsulfat (SDS) ein Anstieg des transepidermalen Wasserverlustes (TEWL), Entzündung und Hyperplasie induziert. Die anschließende topische Applikation von Taurin senkte den TEWL in signifikantem Ausmaß. Weiterhin stimulierte Taurin an Hautmodellen in signifikantem Ausmaß die Synthese aller drei Klassen von Barrierelipiden, nämlich Ceramide, Cholesterole und Fettsäuren.

Außerdem wurde überraschend gefunden, dass bei der Inkubation von Hautmodellen mit Taurin cytotoxische und proinflammatorische Vorgänge inhibiert wurden. Insbesondere nahm die Ausschüttung von Interleukin-1-alpha und von Prostaglandin-E2 ab, die Integrität der Keratinozytenmembran stabilisierte sich und die Vitalität der Keratinozyten verbesserte sich.

Epidermales Interleukin-1-alpha (IL-1 alpha) ist ein bedeutendes proinflammatorisches Cytokin, das an der Entwicklung einer Entzündung und an der Hyperproliferation nach einer Natriumdodecylsulfat (SDS)-induzierten Barrierestörung beteiligt ist. IL-1 alpha kann die Ausschüttung weiterer Cytokine induzieren, darunter IL-6 und IL-8, und wird in der gesamten Epidermis exprimiert, wenn durch wiederholte Barrierestörungen eine epidermale Hyperplasie induziert worden ist.

Basierend auf diesen Haut- und Hautmodelluntersuchungen, war es eine Aufgabe der vorliegenden Erfindung, topische Zusammensetzungen zu entwickeln, die die Barrierefunktion der Haut, beispielsweise gegenüber dem transepidermalen Wasserverlust, stärken und gleichzeitig weitere negative Reaktionen der Haut auf Umweltstress vermindern. Eine weitere Aufgabe der vorliegenden Erfindung war es, topische Zusammensetzungen zu entwickeln, die die Barrierefunktion der Haut stärken und gleichzeitig hautberuhigend und/oder entzündungshemmend wirken. Eine weitere Aufgabe der vorliegenden Erfindung war es, topische Zusammensetzungen zu entwickeln, die die Barrierefunktion der Haut stärken und gleichzeitig hautberuhigend und/oder entzündungshemmend wirken, ohne dabei Nebenwirkungen mit sich zu bringen, wie sie beispielsweise bei Entzündungshemmern auf Kortikoidbasis auftreten können. Eine weitere Aufgabe der vorliegenden Erfindung war es, topische Zusammensetzungen zu entwickeln, die die Barrierefunktion der Haut stärken und gleichzeitig ein angenehmes, nicht-okklusives und/oder nicht-klebriges Hautgefühl aufweisen. Eine weitere Aufgabe der vorliegenden Erfindung war es, topische Zusammensetzungen zu entwickeln, die die Barrierefunktion der Haut mit länger anhaltender Wirkung stärken als die Zusammensetzungen des Standes der Technik. Eine weitere Aufgabe der vorliegenden Erfindung war es, kosten- und herstellungsoptimierte topische Zusammensetzungen zu entwickeln, die die Barrierefunktion der Haut stärken.

Überraschenderweise hat sich gezeigt, dass die Regeneration der Hautbarrierefunktion nach SDS-Schädigung bei Anwendung von Taurin (2-Aminoethansulfonsäure) signifikant beschleunigt wird. Überraschenderweise wurde festgestellt, dass Taurin an Hautmodellen in signifikantem Ausmaß die Synthese aller drei Klassen von Barrierelipiden, nämlich Ceramide, Cholesterole und Fettsäuren, stimulierte. Taurin steigert die Spiegel an Ceramiden, Cholesterol und freien Fettsäuren in gleichem Ausmaß. Diese Eigenschaft ist von zentraler Bedeutung, denn es gilt das korrekte Mischungsverhältnis der drei Barrierelipide beizubehalten (M.M. Man et al. Optimization of physiological lipid mixtures for barrier repair, J. Invest. Dermatol. 106, 1096-101, 1996). Der Zustand trockener Haut, empfindlicher Haut sowie der Altershaut wird nachhaltig verbessert. Taurin ist insbesondere auch zur Behandlung der atopischen Dermatitis geeignet. Im Gegensatz zu den bisher im Stand der Technik bekannten Barrierelipid-haltigen Hautbehandlungsmitteln resultiert die erfindungsgemäße Verwendung von Taurin zur Stimulierung der epidermalen Barrierelipidsynthese in einer Bereitstellung der Barrierelipide über den sekretorischen Mechanismus der Keratinozyten. Auf diese Weise werden die Barrierelipide korrekt enzymatisch prozessiert und funktionell in die multilamellaren Lipidmembranen eingebaut. Die Barrierefunktion wird auf diese Weise dauerhaft verbessert. Der Effekt ist insbesondere noch vorhanden, wenn das Produkt für einige Tage nicht mehr angewendet wird. Weiterhin bietet der Einsatz von Taurin gegenüber den teuren synthetischen Ceramiden ökonomische Vorteile. Weitere ökonomische und verfahrenstechnische Vortile ergeben sich, da Taurin einfach in die Wasserphase eingearbeitet werden kann, während (synthetische) Barrierelipide aufgrund ihres hohen Schmelzpunktes nur unter (kostensteigerndem) Energieeinträg verarbeitet werden können.

Weiterhin wurde überraschend festgestellt, dass Taurin auf biochemischer Ebene die durch anionische Tenside induzierte Freisetzung von Entzündungsmediatoren inhibiert und die zelluläre Vitalität und Integrität deutlich verbessert. Insbesondere der Effekt auf Prostaglandin E2 war für den Fachmann unerwartet, da die chemische Struktur von Taurin keine Ähnlichkeiten zu bekannten NSAIDs (non-steroidal anti-inflammatory drugs) zeigt.

Gegenstand der vorliegenden Erfindung ist die nicht-therapeutische kosmetische Verwendung von Taurin zur Stimulierung der epidermalen Synthese von Barrierelipiden. Ein weiterer Gegenstand der vorliegenden Erfindung ist die nicht-therapeutische kosmetische Verwendung von Taurin zur Regeneration der Hautbarrierefunktion. Ein weiterer Gegenstand der vorliegenden Erfindung ist die nicht-therapeutische kosmetische Verwendung von Taurin zur Beschleunigung der Regeneration der Hautbarrierefunktion. Ein weiterer Gegenstand der vorliegenden Erfindung ist die nicht-therapeutische kosmetische Verwendung von Taurin zur Verbesserung des Erscheinungsbildes trockener Haut. Ein weiterer Gegenstand der vorliegenden Erfindung ist die nicht-therapeutische kosmetische Verwendung von Taurin zur Verbesserung der Ausstrahlung der Haut. Ein weiterer Gegenstand der vorliegenden Erfindung ist die nicht-therapeutische kosmetische Verwendung von Taurin zur Pflege trockener und rissiger Lippen. Ein weiterer Gegenstand der vorliegenden Erfindung ist die nicht-therapeutische kosmetische Verwendung von Taurin zur Stimulierung der epidermalen Synthese von Barrierelipiden, zur Regeneration der Hautbarrierefunktion, zur Beschleunigung der Regeneration der Hautbarrierefunktion, zur Verbesserung des Erscheinungsbildes trockener Haut, zur Verbesserung der Ausstrahlung der Haut und/oder zur Pflege trockener und rissiger Lippen und gleichzeitig zur Hautberuhigung und/oder Entzündungshemmung. Ein weiterer Gegenstand der vorliegenden Erfindung ist die Verwendung von Taurin zur Herstellung eines therapeutischen Hautbehandlungsmittels zur Stimulierung der epidermalen Synthese von Barrierelipiden und gegebenenfalls gleichzeitig zur Hautberuhigung und/oder Entzündungshemmung. Ein weiterer Gegenstand der vorliegenden Erfindung ist die Verwendung von Taurin zur Herstellung eines therapeutischen Hautbehandlungsmittels zur Regeneration der Hautbarrierefunktion und gegebenenfalls gleichzeitig zur Hautberuhigung und/oder Entzündungshemmung. Ein weiterer Gegenstand der vorliegenden Erfindung ist die Verwendung von Taurin zur Herstellung eines therapeutischen Hautbehandlungsmittels zur Behandlung der atopischen Dermatitis.

Erfindungsgemäß verwendete kosmetische oder therapeutische Hautbehandlungsmittel enthalten Taurin in einer Menge von 0,001 - 5,0 Gew.-%, bevorzugt 0,05 - 3,0 Gew.-% und besonders bevorzugt 0,1 - 1,0 Gew.-%, jeweils bezogen auf das Gewicht der gesamten Zusammensetzung.

Vorteilhafterweise liegen die erfindungsgemäß verwendeten kosmetischen oder therapeutischen Hautbehandlungsmittel in Form einer flüssigen, fließfähigen oder festen Öl-in-Wasser-Emulsion, Wasser-in-Öl-Emulsion, Mehrfach-Emulsion, insbesondere einer Öl-in-Wasser-in-Öl- oder Wasser-in-Öl-in-Wasser-Emulsion, Makroemulsion, Miniemulsion, Mikroemulsion, PIT-Emulsion, Nanoemulsion, Pickering-Emulsion, Hydrodispersion, eines Hydrogels, eines Lipogels, einer ein- oder mehrphasigen Lösung, eines Schaumes, eines Puders oder einer Mischung mit mindestens einem als medizinischen Klebstoff geeigneten Polymer vor. Die Mittel können auch in wasserfreier Form, wie beispielsweise einem Öl oder einem Balsam, dargereicht werden. Hierbei kann der Träger ein pflanzliches oder tierisches Öl, ein Mineralöl, ein synthetisches Öl oder eine Mischung solcher Öle sein.

In einer erfindungsgemäß besonders bevorzugt verwendeten Ausführungsform liegen die Hautbehandlungsmittel als Mikroemulsion vor. Unter Mikroemulsionen werden im Rahmen der Erfindung neben den thermodynamisch stabilen Mikroemulsionen auch die sogenannten "PIT"-Emulsionen verstanden. Bei diesen Emulsionen handelt es sich um Systeme mit den 3 Komponenten Wasser, Öl und Emulgator, die bei Raumtemperatur als Öl-in-Wasser-Emulsion vorliegen. Beim Erwärmen dieser Systeme bilden sich in einem bestimmten Temperaturbereich (als Phaseninversiontemperatur oder "PIT" bezeichnet) Mikroemulsionen aus, die sich bei weiterer Erwärmung in Wasser-in-Öl-Emulsionen umwandeln. Beim anschließenden Abkühlen werden wieder O/W-Emulsionen gebildet, die aber auch bei Raumtemperatur als Mikroemulsionen oder als sehr feinteilige Emulsionen mit einem mittleren Teilchendurchmesser unter 400 nm und insbesondere von etwa 100-300 nm, vorliegen. Erfindungsgemäß können solche Mikro- oder "PIT"-Emulsionen bevorzugt sein, die einen mittleren Teilchendurchmesser von etwa 200 nm aufweisen.

In der Ausführungsform als Emulsion enthalten die erfindungsgemäßen Zusammensetzungen mindestens eine oberflächenaktive Substanz als Emulgator oder Dispergiermittel. Geeignete Emulgatoren sind beispielsweise Anlagerungsprodukte von 4 bis 30 Mol Ethylenoxid und/oder 0 bis 5 Mol Propylenoxid an lineare C₈-C₂₂-Fettalkohole, an C₁₂-C₂₂-Fettsäuren und an C₈-C₁₅-Alkylphenole, C₁₂-C₂₂-Fettsäuremono- und -diester von Anlagerungsprodukten von 1 bis 30 Mol Ethylenoxid an C₃-C₆-Polyole, insbesondere an Glycerin, Ethylenoxid- und Polyglycerin-Anlagerungsprodukte an Methylglucosid-Fettsäureester, Fettsäurealkanolamide und Fettsäureglucamide, C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind, Gemische aus Alkyl-(oligo)-glucosiden und Fettalkoholen, z. B. die im Handel erhältlichen Produkte Montanov^{®} 68, Montanov^{®} 202 oder Montanov^{®} L, Anlagerungsprodukte von 5 bis 60 Mol Ethylenoxid an Rizinusöl und gehärtetes Rizinusöl, Partialester von Polyolen mit 3-6 Kohlenstoffatomen mit gesättigten C₈-C₂₂-Fettsäuren, Sterole (Sterine), insbesondere Cholesterol, Lanosterol, Beta-Sitosterol, Stigmasterol, Campesterol und Ergosterol sowie Mykosterole, Phospholipide, vor allem Glucose-Phospolipide, Fettsäureester von Zuckern und Zuckeralkoholen wie Sorbit, Polyglycerine und Polyglycerinderivate, bevorzugt Polyglyceryl-2-di-polyhydroxystearat (Handelsprodukt Dehymuls^{®} PGPH) und Polyglyceryl-3-diisostearat (Handelsprodukt Lameform^{®} TGI) sowie lineare und verzweigte C₈-C₃₀-Fettsäuren und deren Na-, K-, Ammonium-, Ca-, Mg- und Zn - Salze.

Die erfindungsgemäß verwendeten Hautbehandlungsmittel enthalten die Emulgatoren bevorzugt in Mengen von 0,1 bis 25 Gew.-%, insbesondere 0,5 - 15 Gew.-%, bezogen auf das gesamte Mittel.

In einer besonders bevorzugt verwendeten Ausführungsform ist mindestens ein nichtionischer Emulgator mit einem HLB-Wert von 8 und darunter enthalten. Derart geeignete Emulgatoren sind beispielsweise Verbindungen der allgemeinen Formel R¹ - O - R², in der R¹ eine primäre lineare Alkyl-, Alkenyl- oder Acylgruppe mit 20 - 30 C-Atomen und R² Wasserstoff, eine Gruppe mit der Formel -(CₙH₂ₙO)ₓ-H mit x = 1 oder 2 und n = 2 - 4 oder eine Polyhydroxyalkylgruppe mit 4 - 6 C-Atomen und 2 - 5 Hydroxylgruppen ist. Weitere bevorzugt geeignete Emulgatoren mit einem HLB-Wert von 8 und darunter sind die Anlagerungsprodukte von 1 oder 2 Mol Ethylenoxid oder Propylenoxid an Behenylalkohol, Erucylalkohol, Arachidylalkohol oder auch an Behensäure oder Erucasäure. Bevorzugt eignen sich auch die Monoester von C₁₆-C₃₀-Fettsäuren mit Polyolen wie z. B. Pentaerythrit, Trimethylolpropan, Diglycerin, Sorbit, Glucose oder Methylglucose. Beispiele für solche Produkte sind z. B. Sorbitanmonobehenat oder Pentaerythrit-monoerucat.

Weitere geeignete Zusatzstoffe sind Verdickungsmittel, z. B. natürliche und synthetische Tone und Schichtsilikate wie Bentonit, Hectorit, Montmorillonit oder Laponite^{®}, oder anionische Polymere aus Acrylsäure, Methacrylsäure, Crotonsäure, Maleinsäureanhydrid und 2-Acrylamido-2-methylpropansulfonsäure, wobei die sauren Gruppen ganz oder teilweise als Natrium-, Kalium-, Ammonium-, Mono- oder Triethanolammonium-Salz vorliegen können und wobei mindestens ein nichtionisches Monomer enthalten sein kann. Bevorzugte nichtionogene Monomere sind Acrylamid, Methacrylamid, Acrylsäureester, Methacrylsäureester, Vinylpyrrolidon, Vinylether und Vinylester. Bevorzugte anionische Copolymere sind Acrylsäure-Acrylamid-Copolymere sowie insbesondere Polyacrylamidcopolymere mit Sulfonsäuregruppen-haltigen Monomeren. Diese Copolymere können auch vernetzt vorliegen. Geeignete Handelsprodukte sind Sepigel^{®}305, Simulgel^{®}600, Simulgel^{®} NS, Simulgel^{®} EG und Simulgel^{®} EPG der Firma SEPPIC. Weitere besonders bevorzugte anionische Homo- und Copolymere sind unvernetzte und vernetzte Polyacrylsäuren. Solche Verbindungen sind zum Beispiel die Handelsprodukte Carbopol^{®}. Ein besonders bevorzugtes anionisches Copolymer enthält als Monomer zu 80 - 98 % eine ungesättigte, gewünschtenfalls substituierte C₃₋₆-Carbonsäure oder ihr Anhydrid sowie zu 2 - 20 % gewünschtenfalls substituierte Acrylsäureester von gesättigten C₁₀₋₃₀-Carbonsäuren, wobei das Copolymer mit den vorgenannten Vernetzungsagentien vernetzt sein kann. Entsprechende Handelsprodukte sind Pemulen^{®} und die Carbopol^{®}-Typen 954, 980, 1342 und ETD 2020 (ex B.F. Goodrich).

Geeignete nichtionische Polymere sind beispielsweise Polyvinylalkohole, die teilverseift sein können, z. B. die Handelsprodukte Mowiol^{®} sowie VinylpyrrolidonNinylester-Copolymere und Polyvinylpyrrolidone, die z. B. unter dem Warenzeichen Luviskol^{®} (BASF) vertrieben werden.

Die erfindungsgemäß verwendeten Hautbehandlungsmittel enthalten bevorzugt Fettstoffe, die ausgewählt sind aus Ölen, insbesondere pflanzlichen Ölen, wie Sonnenblumenöl, Olivenöl, Sojaöl, Rapsöl, Mandelöl, Jojobaöl, Orangenöl, Weizenkeimöl, Pfirsichkernöl und den flüssigen Anteilen des Kokosöls, Lanolin und seinen Derivaten, flüssigen Paraffinölen, Isoparaffinölen und synthetischen Kohlenwasserstoffen, Di-n-alkylethern mit insgesamt 12 bis 36 C-Atomen, z. B. Di-n-octylether und n-Hexyl-n-octylether, Fettsäuren, besonders linearen und/oder verzweigten, gesättigten und/oder ungesättigten C₈₋₃₀-Fettsäuren, Fettalkoholen, besonders gesättigten, ein- oder mehrfach ungesättigten, verzweigten oder unverzweigten Fettalkoholen mit 4 - 30 Kohlenstoffatomen, die mit 1 - 75, bevorzugt 5 - 20 Ethylenoxid-Einheiten ethoxyliert und/oder mit 3 - 30, bevorzugt 9 - 14 Propylenoxid-Einheiten propoxyliert sein können, Esterölen, das heißt Estern von C₆-₃₀-Fettsäuren mit C₂₋₃₀-Fettalkoholen, Hydroxycarbonsäurealkylestern, Dicarbonsäureestern wie Di-n-butyladipat sowie Diolestern wie Ethylenglykoldioleat oder Propylenglykoldi(2-ethylhexanoat), symmetrischen, unsymmetrischen oder cyclischen Estern der Kohlensäure mit Fettalkoholen, z. B. Glycerincarbonat oder Dicaprylylcarbonat (Cetiol^{®} CC), Mono,- Di- und Trifettsäureestern von gesättigten und/oder ungesättigten linearen und/oder verzweigten Fettsäuren mit Glycerin, die mit 1 - 10, bevorzugt 7 - 9 Ethylenoxid-Einheiten ethoxyliert sein können, z. B. PEG-7 Glyceryl Cocoate, Wachsen, insbesondere Insektenwachsen, Pflanzenwachsen, Fruchtwachsen, Ozokerit, Mikrowachsen, Ceresin, Paraffinwachsen, Triglyceriden gesättigter und gegebenenfalls hydroxylierter C₁₆₋₃₀-Fettsäuren, z. B. gehärteten Triglyceridfetten, Phospholipiden, beispielsweise Sojalecithin, Ei-Lecithin und Kephalinen, Siliconverbindungen, ausgewählt aus Decamethylcyclopentasiloxan, Dodecamethylcyclohexasiloxan und Siliconpolymeren, die gewünschtenfalls quervernetzt sein können, z. B. Polydialkylsiloxanen, Polyalkylarylsiloxanen, ethoxylierten und/oder propoxylierten Polydialkylsiloxanen mit der früheren INCI-Bezeichnung Dimethicone Copolyol, sowie Polydialkylsiloxanen, die Amin- und/oder Hydroxy-Gruppen enthalten, bevorzugt Substanzen mit den INCI-Bezeichnungen Dimethiconol, Amodimethicone oder Trimethylsilylamodimethicone.

Die Einsatzmenge der Fettstoffe beträgt 0,1 - 50 Gew.%, bevorzugt 0,1 - 20 Gew.% und besonders bevorzugt 0,1 - 15 Gew.%, jeweils bezogen auf das gesamte Hautbehandlungsmittel.

Weitere geeignete Zusatzstoffe sind Antioxidantien, Konservierungsmittel, Lösungsmittel wie Ethanol, Isopropanol, Ethylenglykol, Propylenglykol, Propylenglykolmonoethylether, Glycerin und Diethylenglykol, Adsorbentien und Füllstoffe, wie Talkum und Veegum^{®}, Parfümöle, Pigmente sowie Farbstoffe zum Anfärben des Mittels, Substanzen zur Einstellung des pH-Wertes, Komplexbildner wie EDTA, NTA, β-Alanindiessigsäure und

Phosphonsäuren, Treibmittel wie Propan-Butan-Gemische, Pentan, Isopentan, Isobutan, N₂O, Dimethylether, CO₂ und Luft.

### Experimentelle Untersuchungen

**Tab. 1: Einfluss von Taurin auf die epidermale Barrierefunktion nach repetitiver SDS-Schädigung in vivo (SDS = Natriumdodecylsulfat)**

| | Mittelwert [arbitrary units] | Standardabweichung |
|---|---|---|
| Unbehandelt (VAR3) | 2908610 (100 %) | 1281065 (44) |
| Vehikel (VAR 1) | 1816829 (62,5 %) | 567339 (19,5) |
| Vehikel + 1% Taurin (VAR2) | 881932 (30,3 %) | 236546 (8,1) |

Die Barriere der Haut wurde durch okklusive Applikation von 0.75% SDS geschädigt. Anschließend wurden Öl-in-Wasser-Emulsionen mit 1,5 Gew.-% Behenylalkohol und 2,75 Gew.-% Arachidylalkohol (VAR1: 0 Gew.-% Taurin; VAR2: 1 Gew.-% Taurin) appliziert, oder die Haut verblieb unbehandelt (VAR3). Diese Behandlung wurde zweimal täglich an 5 Tagen durchgeführt. Der transepidermale Wasserverlust (TEWL) wurde jeweils vor der SDS-Schädigung mit einem TEWAMETER (Courage & Khazaka, Köln, Germany) bestimmt. Die Daten wurden auf die t₀-Werte normiert und ein dimensionsloser Wert "area under the curve" errechnet (siehe Tabelle 1). Die statistische Analyse der Daten basiert auf dem gepaarten Wilcoxon-Test. Die Daten repräsentieren die Mittelwerte (n=18).

**Tab. 2: Einfluss von Taurin auf Irritationsmarker in vitro**

| | Vitalität [%] +/- SEM | LDH- Freisetzung [OD 492nm] +/- SEM | IL-1alpha- Freisetzung [pg/ml] +/- SEM | PGE2-Freisetzung [ng/ml] +/- SEM |
|---|---|---|---|---|
| A: unbehandelt | 100 +/- 4 | 0,22 +/- 0,02 | 15 +/- 1 | 0,84 +/- 0,17 |
| B: 0,18% SDS | 53 +/- 12 | 2,21 +/- 0,35 | 395 +/- 106 | 6,69 +/- 0,68 |
| C: 0,1% Triamcinolonacetonid | 86 +/- 8 | 1,19 +/- 0,16 | 292 +/- 26 | 0,30 +/- 0,05 |
| D: Vehikel | 66 +/- 7 | 1,95 +/- 0,25 | 387 +/- 55 | 1,40 +/- 0,29 |
| E: Vehikel + 1% Taurin | 82 +/- 2 | 1,30 +/- 0,15 | 288 +/- 37 | 0,83 +/- 0,05 |

Epidermismodelle wurden 1 Stunde lang topisch mit einer wässrigen Lösung von 0,18% SDS in Wasser inkubiert. Diese Behandlung resultierte erwartungsgemäß in einer deutlichen Verminderung der zellulären Vitalität, einer gesteigerten Freisetzung von LDH (Lactatdehydrogenase), Interleukin 1-alpha (IL-1alpha) und Prostaglandin E2 (PGE2). Anschließend wurden die Epidermismodelle abgespült und für weitere 24h unter topischer Applikation folgender Emulsionen inkubiert: C: Kortikoid-ratiopharm® Creme (0.1 Gew.-% Triamcinolonacetonid); D: 1,5 Gew.-% Behenylalkohol + 2,75 Gew.-% Arachidylalkohol; E: 1,5 Gew.-% Behenylalkohol + 2,75 Gew.-% Arachidylalkohol +1 Gew.-% Taurin. Zur Kontolle wurden Epidermismodelle unbehandelt kultiviert (A) oder verblieben nach Abspülung der SDS-Lösung unbehandelt (B). Die Daten repräsentieren die Mittelwerte +/-SEM (n=3).

### Einfluss von Taurin auf die epidermale Synthese von Barrierelipiden

Epidermismodelle wurden 12 Tage lang kultiviert. Nach 12 Tagen (t = 0) wurden die individuell entwickelten sogenannten Barrierelipide (Ceramide, Cholesterol, Fettsäuren, Cholesterolsulfate und Cholesterolester) quantifiziert. Anschließend wurde ein Teil der Epidermismodelle 5 Tage lang ohne Zusatz von Taurin weiterkultiviert. Ein anderer Teil der Epidermismodelle wurde 5 Tage lang ohne Zusatz von 0,3 mM Taurin weiterkultiviert. Nach insgesamt 17 Tagen Kultivierungsdauer (t = 5) wurden erneut die Barrierelipide quantifiziert. Es zeigte sich, dass der Zusatz von Taurin zu einer Steigerung der epidermalen Synthese von Barrierelipiden führte (siehe Tabelle 3). Die in Tab. 3 gezeigten Unterschiede zu t = 5 waren statistisch signifikant (p<0,05).

**Tabelle 3: Einfluss von Taurin auf die epidermale Synthese von Barrierelipiden (Angaben der Menge an Barrierelipiden in Gew.-%, bezogen auf den Gesamtlipidgehalt der Epidermismodelle, Mittelwerte aus n = 4 Messungen (± Standardabweichung))**

| | Ceramide | Cholesterol | Fettsäuren | Cholesterol- sulfat | Cholesterol-ester |
|---|---|---|---|---|---|
| t = 0 | 5,6 (± 1,6) | 5,6 (± 1,2) | 3,2 (± 1,0) | 1,4 (± 0,3) | 0,6 (± 0,1) |
| t = 5 (ohne Taurin) | 7,1 (± 1,8) | 9,9 (± 1,0) | 3,5 (± 1,1) | 0,6 (± 0,1) | 0,9 (± 0,3) |
| t = 5 (0,3 mM Taurin) | 11.7 (± 1,6) | 11,0 (± 1,2) | 5,7 (± 1,5) | 1,0 (± 0,2) | 1,6 (± 0,2) |

Die folgenden Beispiele stellen erfindungsgemäß verwendete therapeutische und/oder kosmetische Hautbehandlungsmittel dar und sollen die Erfindung erläutern, ohne sie hierauf zu beschränken.

### 1. Oel-in-Wasser-Emulsionen

### 1. Beispielserie:

| | 1.1.1 | 1.1.2 | 1.1.3 |
|---|---|---|---|
| Distelöl | 3,00 | 3,00 | 3,00 |
| Myritol318 | 5,00 | 5,00 | 5,00 |
| Novata AB | 2,00 | 2,00 | 2,00 |
| Lanette 22 | 1,00 | 1,00 | 1,00 |
| Cutina MD-V | 2,00 | 2,00 | 2,00 |
| Stenol 1618 | 1,00 | 1,00 | 1,00 |
| Isopropylstearat | 4,00 | 4,00 | 4,00 |
| Cetiol SB 45 | 2,00 | 2,00 | 2,00 |
| Baysilone-Öl M 350 | 1,00 | 1,00 | 1,00 |
| Controx KS | 0,05 | 0,05 | 0,05 |
| Propylparaben | 0,20 | 0,20 | 0,20 |
| Dow Corning 1501 Fluid | 1,00 | 1,00 | 1,00 |
| Dry Flo Plus | 1,00 | 1,00 | 1,00 |
| TiO₂ | 0,50 | 0,50 | 0,50 |
| Hexandiol | 6,00 | 3,00 | |
| Propylenglycol | 5,00 | 5,00 | 5,00 |
| Glycerin | 5,00 | 3,00 | 3,00 |
| Methylparaben | 0,20 | 0,20 | 0,20 |
| Tego Carbomer 140 | 0,40 | 0,40 | 0,40 |
| DSH-CN | 5,00 | 5,00 | 5,00 |
| Algenextrakt | 1,00 | 0,50 | 0,10 |
| Taurin | 1,00 | 0,50 | 0,10 |
| Parfum | 0,10 | 0,10 | 0,10 |
| Aqua | ad 100 | ad 100 | ad 100 |

### 2. Beispielserie:

| | 1.2.1 | 1.2.2 | 1.2.3 |
|---|---|---|---|
| Cetiol SN | 4,00 | 4,00 | 4,00 |
| Mineralöl | 6,00 | 6,00 | 6,00 |
| Cutina CBS | 2,00 | 2,00 | 2,00 |
| Edenor L2 SM | 1,50 | 1,50 | 1,50 |
| Eumulgin B3 | 1,00 | 1,00 | 1,00 |
| Baysilone-Öl M 350 | 1,00 | 1,00 | 1,00 |
| Tocopherylacetat | 0,50 | 0,50 | 0,50 |
| Propylparaben | 0,30 | 0,30 | 0,30 |
| Mandelöl | 2,00 | 2,00 | 2,00 |
| Pemulen TR 1 | 0,27 | 0,27 | 0,27 |
| Glycerin | 5,00 | 3,00 | 3,00 |
| Milchsäure 80% | 0,26 | 0,26 | 0,26 |
| Propylenglycol | 5,00 | 5,00 | 5,00 |
| Methylparaben | 0,30 | 0,30 | 0,30 |
| Phenoxyethanol | 0,90 | 0,90 | 0,90 |
| Panthenol | 0,50 | - | - |
| Seanergilium | 1,00 | - | - |
| Natriumchlorid | 0,05 | 0,05 | 0,05 |
| Sepigel 305 | 0,50 | 0,50 | 0,50 |
| Silk Protein COS 152/21 A | 0,25 | 0,25 | 0,25 |
| Keltrol SF | 0,20 | 0,20 | 0,20 |
| Taurin | 1,00 | 0,50 | 0,10 |
| Parfum | 0,30 | 0,30 | 0,30 |
| Aqua | ad 100 | ad 100 | ad 100 |

### 3. Beispielserie:

| | 1.3.1 | 1.3.2 | 1.3.3 |
|---|---|---|---|
| Lipoid S 75-3 | 0,50 | 0,50 | 0,50 |
| Isopropylstearat | 4,00 | 4,00 | 4,00 |
| Cetiol B | 2,00 | 2,00 | 2,00 |
| Tocopherylacetat | 0,50 | 0,50 | 0,50 |
| Cutina MD-V | 1,00 | 1,00 | 1,00 |
| Lanette 22 | 2,00 | 2,00 | 2,00 |
| Baysilone-Öl M 350 | 0,50 | 0,50 | 0,50 |
| Propylparaben | 0,20 | 0,20 | 0,20 |
| Dow Corning 9040 | 1,00 | 1,00 | 1,00 |
| Glycerin | 4,50 | 3,00 | 3,00 |
| Hexandiol | 6,00 | 3,00 | |
| Methylparaben | 0,20 | 0,20 | 0,20 |
| Tego Carbomer 140 | 0,30 | 0,30 | 0,30 |
| DSH-CN | 5,00 | 5,00 | 5,00 |
| Algenextrakt | 1,00 | | |
| Photosomes | 0,20 | 0,20 | 0,20 |
| Lipochroman-6 | 0,01 | 0,01 | 0,01 |
| Propylenglycol | 5,00 | 5,00 | 5,00 |
| Matrixyl | 3,00 | | |
| Matrixyl 3000 | | 3,00 | |
| Simulgel NS | 1,50 | 1,50 | 1,50 |
| TiO₂ | 0,50 | 0,50 | |
| Taurin | 1,00 | 0,50 | 0,10 |
| Parfum | 0,35 | 0,35 | 0,35 |
| Aqua | ad 100 | ad 100 | ad 100 |

### 4. Beispielserie:

| | 1.4.1 | 1.4.2 | 1.4.3 | 1.4.4 | 1.4.5 |
|---|---|---|---|---|---|
| Montanov 68 | 5,00 | 5,00 | 5,00 | 5,00 | 5,00 |
| Myritol318 | 5,00 | 5,00 | 5,00 | 5,00 | 5,00 |
| Cetiol SB 45 | 0,50 | 0,50 | 0,50 | 0,50 | 0,50 |
| Novata AB | 2,00 | 2,00 | 2,00 | 2,00 | 2,00 |
| Stenol 16/18 | 1,00 | 1,00 | 1,00 | 1,00 | 1,00 |
| Baysilone-Öl M 350 | 0,50 | 0,50 | 0,50 | 0,50 | 0,50 |
| Tocopherylacetat | 0,50 | 0,50 | 0,50 | 0,50 | 0,50 |
| Controx KS | 0,25 | 0,25 | 0,25 | 0,25 | 0,25 |
| Parsol SLX | | 4,00 | 4,00 | | |
| Parsol 340 | | | | 5,00 | |
| Uvinul MBC 95 | 2,00 | | | | |
| Parsol 1789 | 1,00 | 1,80 | 1,80 | | |
| Propylparaben | 0,20 | 0,20 | 0,20 | 0,20 | 0,20 |
| Tego Carbomer 140 | 0,50 | 0,50 | 0,50 | 0,50 | 0,50 |
| Hexandiol | 6,00 | 6,00 | 3,00 | 6,00 | |
| Talkum Pharma G | 0,50 | 0,50 | 0,50 | 0,50 | 0,50 |
| Methylparaben | 0,20 | 0,20 | 0,20 | 0,20 | 0,20 |
| Glycerin | 4,50 | 4,50 | 3,00 | 4,50 | 3,00 |
| Dry Flo Plus | 1,00 | 1,00 | 1,00 | 1,00 | 1,00 |
| Natipide II PG | 1,00 | 1,00 | 1,00 | 1,00 | 1,00 |
| Gatuline R/C | 2,00 | 2,00 | 2,00 | 2,00 | 2,00 |
| Silk Protein COS 152/21 A | 5,00 | 5,00 | 5,00 | 5,00 | 5,00 |
| DSH-CN | 2,00 | 2,00 | 2,00 | 2,00 | 2,00 |
| Trilon A | 0,10 | 0,10 | 0,10 | 0,10 | 0,10 |
| Phenoxyethanol | 0,40 | 0,40 | 0,40 | 0,40 | 0,40 |
| Taurin | 1,00 | 0,50 | 0,10 | 1,00 | 1,00 |
| Parfum | 0,40 | 0,40 | 0,40 | 0,40 | 0,40 |
| Aqua | ad 100 | ad 100 | ad 100 | ad 100 | ad 100 |

### 5. Beispielserie:

| | 1.5.1 | 1.5.2 | 1.5.3 |
|---|---|---|---|
| Montanov L | 3,00 | 3,00 | 3,00 |
| Cetiol B | 6,00 | 6,00 | 6,00 |
| Myritol 318 | 3,00 | 3,00 | 3,00 |
| Stenol 16/18 | 1,00 | 1,00 | 1,00 |
| Cutina MD-V | 0,50 | 0,50 | 0,50 |
| Baysilone-Öl M 350 | 0,50 | 0,50 | 0,50 |
| Propylparaben | 0,20 | 0,20 | 0,20 |
| Dow Corning 245 Fluid | 1,50 | 1,50 | 1,50 |
| Glycerin | 5,00 | 5,00 | 5,00 |
| Karion F | 3,00 | 3,00 | 3,00 |
| Methylparaben | 0,20 | 0,20 | 0,20 |
| Dry Flo Plus | 0,50 | 0,50 | 0,50 |
| Tego Carbomer 140 | 0,10 | 0,10 | 0,10 |
| DSH-CN | 2,00 | 2,00 | 2,00 |
| Seanergilium | 0,50 | 0,50 | 0,50 |
| Simulgel NS | 1,00 | 1,00 | 1,00 |
| Taurin | 1,00 | 0,50 | 0,10 |
| Parfum | 0,20 | 0,20 | 0,20 |
| Aqua | ad 100 | ad 100 | ad 100 |

### 2. Wasser-in-Oel-Emulsion

| | 2.1.1 | 2.1.2 | 2.1.3 |
|---|---|---|---|
| Lameform TGI | 3,00 | 3,00 | 3,00 |
| Elfacos ST 9 | 0,50 | 0,50 | 0,50 |
| Microcrystalline Wax | 3,00 | 3,00 | 3,00 |
| Bis-Diglyceryl Polyacyladipate-2 | 1,00 | 1,00 | 1,000 |
| Paraffinöl | 8,00 | 8,00 | 8,00 |
| Vaseline | 2,00 | 2,00 | 2,00 |
| Tocopherylacetat | 2,00 | 2,00 | 2,00 |
| Methylparaben | 0,30 | 0,30 | 0,30 |
| Propylparaben | 0,30 | 0,30 | 0,30 |
| Isopropylisostearat | 8,00 | 8,00 | 8,00 |
| Glycerin | 5,00 | 3,00 | 3,00 |
| Mg-Sulfat | 0,50 | 0,50 | 0,50 |
| Taurin | 1,00 | 0,50 | 0,10 |
| Milchsäure 80%ig | 0,56 | 0,56 | 0,56 |
| Algenextrakt | 1,00 | | |
| Panthenol | 0,50 | 1,00 | 0,50 |
| Calendula Officinalis Flower Extract | 0,30 | | |
| Propylenglycol | 0,50 | | |
| Parfum | 0,20 | 0,20 | 0,20 |
| Aqua | ad100 | ad100 | ad100 |

### 3. Reinigungszubereitungen

### 1. Beispielserie: Gesichtswässer

| | 3.1.1 | 3.1.2 | 3.1.3 |
|---|---|---|---|
| Dipropylenglycol | 10,00 | 10,00 | 10,00 |
| Chlorhexidindigluconat | 1,00 | 1,00 | 1,00 |
| Synperonic PE/L 64 | 3,00 | 3,00 | 3,00 |
| D-Panthenol | 0,50 | 0,50 | 0,50 |
| Hydagen CMF | 3,00 | 3,00 | 3,00 |
| PEG-40 Hydrogenated Castor Oil / Trideceth 9 / Propylene Glycol | 0,50 | 0,50 | 0,50 |
| Chlorella Vulgaris Extract | 0,50 | 0,50 | 0,50 |
| Taurin | 1,00 | 0,50 | 0,10 |
| Parfume | 0,20 | 0,20 | 0,20 |
| Aqua | ad 100 | ad 100 | ad 100 |

### 2. Beispielserie: Schäumende Waschgel-Zubereitungen

| | 3.2.1 | 3.2.2 | 3.2.3 |
|---|---|---|---|
| Carbopol ETD 2001 | 1,40 | 1,40 | 1,40 |
| Sorbitol | 2,10 | 2,10 | 2,10 |
| Natriumbenzoat | 0,40 | 0,40 | 0,40 |
| Plantacare 2000UP | 7,50 | 7,50 | 7,50 |
| Cocoamidopropyl betaine | 3,40 | 3,40 | 3,40 |
| Texapon SB 3 | 5,00 | 5,00 | 5,00 |
| Cetiol HE | 0,50 | 0,50 | 0,50 |
| Lamesoft PO 65 | 5,00 | 5,00 | 5,00 |
| Controx KS | 0,05 | 0,05 | 0,05 |
| Ajidew NL 50 | 1,60 | 1,60 | |
| Pantolacton | 1,00 | 1,00 | |
| Trilon B | 0,25 | 0,25 | 0,25 |
| Natriumlactat | 1,80 | | |
| D-Panthenol | 0,50 | 0,50 | 0,50 |
| Taurin | 1,00 | 0,10 | 0,50 |
| Parfum | 0,40 | 0,40 | 0,40 |
| Aqua | ad 100 | ad 100 | ad 100 |

### 3. Beispielserie: Schäumende Waschgel-Zubereitungen

| | 3.3.1 | 3.3.2 | 3.3.3 |
|---|---|---|---|
| Hostapon SCI-65 | 22,50 | 22,50 | 22,50 |
| Talkum | 4,00 | 4,00 | 4,00 |
| Cutina FS 45 | 6,00 | 6,00 | 6,00 |
| Cocoamidopropyl betain 40% | 9,50 | 9,50 | 9,50 |
| Na-benzoat | 0,40 | 0,40 | 0,40 |
| Sorbitol 70% | 1,00 | 1,00 | 1,00 |
| Glycerin 86% | 1,00 | 1,00 | 1,00 |
| D-Panthenol 75 % | 0,13 | 0,13 | 0,13 |
| Taurin | 1,00 | 0,10 | 0,50 |
| Parfum | 0,30 | 0,30 | 0,30 |
| Aqua | ad 100 | ad 100 | ad 100 |

### 4. Beispielserie:

| | 3.4.1 | 3.4.2 | 3.4.3 |
|---|---|---|---|
| Paraffinum Liquidum | 20,00 | 20,00 | 20,00 |
| Hostaphat KW 340 D | 2,50 | 2,50 | 2,50 |
| Cetearyl Alcohol | 1,40 | 1,40 | 1,40 |
| Eumulgin B 1 | 1,70 | 1,70 | 1,70 |
| Tocopherylacetat | 0,25 | 0,25 | 0,25 |
| Propylparaben | 0,20 | 0,20 | 0,20 |
| Glycerin 86% pflanzlich | 2,00 | 2,00 | 2,00 |
| Hexandiol-1,6 | 5,00 | 5,00 | 5,00 |
| Sorbit 70% LS DAB | 1,00 | 1,00 | 1,00 |
| Methylparaben | 0,20 | 0,20 | 0,20 |
| Tego Carbomer 140 | 0,30 | 0,30 | 0,30 |
| Keltrol SF | 0,10 | 0,10 | 0,10 |
| D-Panthenol 75 % | 0,70 | 0,70 | 0,70 |
| Phenoxyethanol, rein | 0,40 | 0,40 | 0,40 |
| Calcium D-Pantothenat | 0,05 | 0,05 | 0,05 |
| Taurin | 1,00 | 0,10 | 0,50 |
| Parfum | 0,25 | 0,25 | 0,25 |
| Aqua | ad 100 | ad 100 | ad 100 |

### 4. Wasser-in-Silicon-Emulsionen

| | 4.1 | 4.2 | 4.3 |
|---|---|---|---|
| Belsil DM 100 | 2,50 | 2,50 | 2,50 |
| Dow Corning 245 Fluid | 25,00 | 25,00 | 25,00 |
| Abil EM 90 | 2,00 | 2,00 | 2,00 |
| Natriumchlorid | 2,00 | 2,00 | 2,00 |
| Parfum | 0,30 | 0,30 | 0,30 |
| Symdiol 68 | 0,30 | 0,30 | 0,30 |
| Phenoxyethanol | 0,40 | 0,40 | 0,40 |
| Taurin | 1,00 | 0,50 | 0,10 |
| Aqua | ad 100 | ad 100 | ad 100 |

### Liste der verwendeten Rohstoffe

| Handelsname | INCI-Bezeichnung | Lieferant/Hersteller |
|---|---|---|
| | | |
| Abil EM 90 | Cetyl PPG/PEG 10/1 Dimethicone | Degussa |
| Ajidew^{®} NL 50 | Sodium PCA | AJINOMOTO |
| Baysilone-Öl M 350 | Dimethicone | GE Bayer Silicones |
| Belsil DM 100 | Dimethicone | Wacker |
| Benecel MP 333 C | HYDROXYPROPYL METHYLCELLULOSE | Hercules |
| Betafin BP 20 | Betaine | Danisco |
| Biopeptide CL | N-Palmitoyl-Gly-His-Lys | Sederma |
| Biopeptide EL | N-Palmitoyl-Val-Gly-Val-Ala-Pro-Gly | Sederma |
| Calmosensine | Acetyl Dipeptide-1 Cetylester | Sederma |
| Carbopol ETD 2001 Cetiol B | Carbomer | Noveon |
| | Dibutyl Adipate | Cognis |
| Cetiol^{®} HE | PEG-7 Glyceryl Cocoate | Cognis |
| Cetiol^{®} SB 45 | Butyrospermum Parkii (Shea Butter) | Cognis |
| Cetiol^{®} SN | Cetearyl Isononanoate | Cognis |
| Controx KS | Tocopherol, Hydrogenated Palm Glycerides Citrate | Cognis |
| Cutina^{®} CBS | Glyceryl Stearate, Cetearyl Alcohol, Cetyl Palmitate, Cocoglycerides | Cognis |
| Cutina FS 45 | Palmitic Acid, Stearic Acid | Cognis |
| Cutina^{®} MD-V | Glyceryl Stearate (vegetal) | Cognis |
| Dow Corning 1501 Fluid | Cyclomethicone, Dimethiconol (83 % Decamethylcyclopentasiloxan, 15 -17 % Dimethiconol) | Dow Corning |
| Dow Corning 9040 | Cyclomethicone/ Dimethicone Crosspolymer | Dow Corning |
| Dow Corning^{®}245 | Cyclomethicone | Dow Corning |
| Dry Flo Plus | Aluminium Starch Octenylsuccinate | National Starch |
| DSH CN | Water, Dimethylsilanol Hyaluronate | Exsymol |
| Edenor L2 SM | Palmitic Acid, Stearic Acid | Cognis |
| Elfacos ST 9 | PEG-45/Dodecyl Glycol Copolymer | Akzo Nobel |
| Eumulgin B 1 | Ceteareth-12 | Cognis |
| Eumulgin B 3 | Ceteareth-30 | Cognis |
| Gatuline R/C | Water, Fagus Sylvatica Extract | Gattefossé |
| Hostaphat^{®} KW 340 D | TRICETEARETH-4 PHOSPHATE | Clariant |
| Hostapon^{®} SCI 65 | Sodium Cocoyl Isethionate (63 - 69 % Aktivsubstanz) | Clariant |
| Hydagen CMF | Water, Chitosan Glycolate | Cognis |
| Karion F | Sorbitol (70 %), Water | Merck |
| Keltrol SF | Xanthan Gum | CP Kelco |
| Lameform TGI | Polyglyceryl-3 Diisostearate | Cognis |
| Lamesoft^{®} PO 65 | Coco-Glucoside, Glyceryl Oleate, Water | Cognis |
| Lanette^{®} 22 | Behenyl Alcohol | Cognis |
| Lipochroman 6 | Dimethylmethoxy Chromanol | Lipotec |
| Lipoid S 75-3 | Water, Hydrogenated Lecithin | Lipoid GmbH |
| Matrixyl | Aqua, Palmitoyl Pentapeptide-3 | Sederma |
| Matrixyl 3000 | Glycerin, Aqua, Butylene Glycol, Carbomer, Polysorbate 20, Palmitoyl Oligopeptide, Palmitoyl Tetrapeptide-1 | Sederma |
| Montanov 202 | Arachidyl Alcohol, Behenyl Alcohol, Arachidyl Glucoside | Seppic |
| Montanov 68 | Cetearyl Alcohol, Cetearyl Glucoside | Seppic |
| Montanov L | C14-22 Alcohols/C12-20 Alkyl Glucoside | Seppic |
| Myritol 318 | Caprylic/Capric Triglyceride | Cognis |
| Natipide II PG | Aqua (Water), Propylene Glycol, Lecithin | Kuhs GmbH |
| Novata AB | Cocoglycerides | Cognis |
| Oleanoline DPG | Dipropylene Glycol, Olea Europaea (Olive) Leaf Extract | Vincience |
| Omega-CH-Aktivator | Tripeptide-1 | GfN |
| Parsol 1789 | Butyl Methoxydibenzoylmethane | Roche |
| Parsol 340 | Octocrylene | Roche |
| Parsol SLX | Polysilicone-15 | Roche |
| Pemulen TR 1 | Acrylates/C10-30 Alkyl Acrylate Cross- polymer | Noveon |
| Photosomes | Aqua (Water), Lecithin, Plancton Extract | AGI Dermatics (Barnet) |
| Plantacare^{®} 2000 UP | Decyl Glucoside, ca. 50 % Aktivsubstanz | Cognis |
| Seanergilium | LAMINARIA DIGITATA EXTRACT, BUTYLENE GLYCOL, METHYLPARABEN | Coletica |
| Sepigel^{®}305 | Polyacrylamide, C₁₃-C₁₄ Isoparaffin, Laureth-7 | SEPPIC |
| Sepivinol R | Polyphenolreicher Extrakt aus Rotwein | Seppic |
| Silk Protein COS 152/21 A | ALCOHOL DENAT.,HYDROLYZED SILK, PEG-8 | Cosmetochem |
| Silymarin Phytosome | Silybum Marianum Extract and Phospholipids | Indena SpA |
| Simulgel NS | Hydroxyethyl Acrylate / Sodium Acryloyl- dimethyl Taurate Copolymer / Squalane / Polysorbate 60 | Seppic |
| Soy Protein Isolate | Isoflavonoidreicher Extrakt aus Soja | Protein Technology International |
| Stenol 16/18 | Cetearyl Alcohol | Cognis |
| Symdiol 68 | 1,2-Octandiol, 1,2-Hexandiol | Symrise |
| Synperonic PE/L 64 | Poloxamer-184 | Uniqema |
| Tego Carbomer 140 | Carbomer | Goldschmidt |
| Texapon^{®} SB 3 | Water, Disodium Laureth Sulfosuccinate, (ca. 38 - 42 % Aktivsubstanz), citric acid | Cognis |
| Trilon A | Trisodium NTA | BASF |
| Trilon B | Tetrasodium EDTA | BASF |
| Uvinul MBC 95 | 4-Methylbenzylidene Campher | BASF |

## Patentansprüche

1. Nicht-therapeutische kosmetische Verwendung von Taurin zur Stimulierung der epidermalen Synthese von Barrierelipiden.

2. Nicht-therapeutische kosmetische Verwendung von Taurin nach Anspruch 1 zur Regeneration der Hautbarrierefunktion.

3. Nicht-therapeutische kosmetische Verwendung von Taurin nach Anspruch 1 zur Beschleunigung der Regeneration der Hautbarrierefunktion.

4. Nicht-therapeutische kosmetische Verwendung von Taurin nach Anspruch 1 zur Verbesserung des Erscheinungsbildes trockener Haut.

5. Nicht-therapeutische kosmetische Verwendung von Taurin nach Anspruch 1 zur Verbesserung der Ausstrahlung der Haut.

6. Nicht-therapeutische Verwendung eines kosmetischen Hautbehandlungsmittels nach einem der Ansprüche 1 - 5 zur Pflege trockener und rissiger Lippen.

7. Nicht-therapeutische kosmetische Verwendung nach einem der Ansprüche 1 - 6 zur Hautberuhigung und/oder Entzündungshemmung.

8. Verwendung von Taurin zur Herstellung eines therapeutischen Hautbehandlungsmittels zur Stimulierung der epidermalen Synthese von Barrierelipiden.

9. Verwendung von Taurin zur Herstellung eines therapeutischen Hautbehandlungsmittels nach Anspruch 8 zur Regeneration der Hautbarrierefunktion.

10. Verwendung von Taurin zur Herstellung eines therapeutischen Hautbehandlungsmittels nach Anspruch 8 oder 9 und gleichzeitig zur Hautberuhigung und/oder Entzündungshemmung.

11. Verwendung von Taurin zur Herstellung eines therapeutischen Hautbehandlungsmittels nach Anspruch 8 zur Behandlung der atopischen Dermatitis.

12. Verwendung gemäß einem der vorstehenden Ansprüche, **dadurch gekennzeichnet, dass** das Taurin in einem Hautbehandlungsmittel in Form einer flüssigen, fließfähigen oder festen Öl-in-Wasser-Emulsion, Wasser-in-Öl-Emulsion, Mehrfach-Emulsion, Makroemulsion, Miniemulsion, Mikroemulsion, PIT-Emulsion, Nanoemulsion, Pickering-Emulsion, Hydrodispersion, eines Hydrogels, eines Lipogels, einer ein- oder mehrphasigen Lösung, eines Schaumes, eines Puders oder einer Mischung mit mindestens einem als medizinischen Klebstoff geeigneten Polymer enthalten ist.
